# EUROPEAN PATENT SPECIFICATION

(11) **EP 3 713 489 B1**
(45) Date of publication and mention of the grant of the patent: **03.04.2024**
(21) Application number: 18865881.9
(22) Date of filing: 12.10.2018
(51) Int. Cl.: A61B 5/157, A61B 5/151, A61B 5/15

(54) **INTEGRATED BLOOD TEST DEVICE**
INTEGRIERTE BLUTTESTVORRICHTUNG
DISPOSITIF DE TEST SANGUIN INTÉGRÉ

(30) Priority: 12.10.2017 AU 2017904127
(43) Date of publication of application: 30.09.2020
(73) Proprietor: Atomo Diagnostics Limited, Leichhardt, NSW 2040 (AU)
(72) Inventor: POSTLE, Will, Leichhardt, NSW 2040 (AU); SUTTON, David, Leichhardt, NSW 2040 (AU); GIJZEL, Martijn, Leichhardt, NSW 2040 (AU); CUSACK, Doug, Leichhardt, NSW 2040 (AU); VAN DE SANDE, Barbara, Leichhardt, NSW 2040 (AU); DAWSON, Benjamin, Leichhardt, NSW 2040 (AU)
(74) Representative: Budde Schou A/S
(86) International application number: PCT/AU2018/051114
(87) International publication number: WO 2019/071323

(56) References cited:
- WO-A1-2011/113114
- WO-A1-2012/048388
- WO-A1-2015/075677
- WO-A2-2007/111651
- US-A1- 2005 186 111
- US-A1- 2007 087 357
- US-A1- 2008 300 508

## Description

### Technical Field

The present invention relates to devices for conducting blood tests using an integrated testing device.

### Background of the Invention

Blood tests have conventionally been conducted by drawing blood, for example by venepuncture, and submitting blood for testing to central pathology facilities. Increasingly, there is a demand for tests to be undertaken and interpreted at the point of care, and for self-tests where the user takes their own blood, submits it to a local test device and reads the test outcome.

Advances in the structure, chemistry and biochemistry of test materials have produced the possibility to extend self- testing to a much wider group of users and conditions, particularly where the potential exists for relatively rapid tests. Tests have been developed to allow for testing for many indications, for example various pathogen, antibodies, blood components, biochemical markers, and other substances.

The present applicant has filed applications directed at various aspects of integrated test units, including for example WO/2012/048388, WO/2011/113114, and WO2015075677.

WO 2012/048388 discloses an assembly for sampling bodily fluid. The assembly has a membrane penetration device that has a membrane penetrating element for penetrating a bodily membrane to release a bodily fluid. The assembly has also a collector that is configured in a collection position to take up the released bodily fluid and retain the fluid for delivery to a test element.

WO 2011/113114 discloses a composite diagnostic system that includes a support member that has a membrane penetration element; a bodily collection point for collection of a bodily fluid released by application of the membrane penetration element to a user's body and a test material positioned in the support member such that in use the bodily fluid is brought into contact with the test material.

WO 2015/075677 discloses an integrated testing device for testing bodily fluids. The device has a test component, a reservoir containing a test fluid and a control vessel.

Other examples of devices that determine the presence of an analyte in a fluid sample include US 2007/0087357, US 2005/0186111 and US 2008/0300508.

US 2007/0087357 discloses devices, methods and kits for conducting an assay to determine the presence or amount of an analyte in a fluid sample. The device has a sample application element and a flow path matrix that facilitates fluidic flow by capillary action.

US 2005/0186111 discloses a self-contained device using a gravitationally encouraged, interrupted downward and programmed flow of fluid to provide rapid confirmatory immunological testing (RCIT) in a point-of-care setting.

US 2008/0300508 discloses medical diagnostics devices and more specifically to a diagnostic patch attachable to a subject's skin for performing a rapid blood test.

There is a need for further refinement in such devices, for example in relation to facilitating self-testing. Self-testing presents a very specific set of design challenges, relative to tests intended for professional or regular users. The user will often not have regular, or even any, experience in conducting such tests. Thus, the risk that errors will be made, which in turn undermine the quality of the outcome, is significant.

It is an object of the present invention to provide integrated testing devices which are reliable and reduce the risk of errors by the user.

### Summary of the Invention

In first broad form, the present invention provides a device in which test fluid and blood are delivered to a test material by a single actuator.

According to one aspect, the present invention provides an integrated blood test unit including a blood collection unit, a test component, a reservoir containing test fluid, and an actuator, wherein operatively activation of the actuator causes movement of the blood collection unit to deliver blood to the test component, and activation of the actuator further causes the test fluid to be released from the reservoir in order to allow the test fluid to contact the test component.

Suitable implementations of the present invention accordingly allow for easily used devices, particularly for self test applications, which facilitate

### Brief Description of the Drawings

Illustrative embodiments of the present invention will now be described with reference to the accompanying drawings, in which:
Figure 1 shows an isometric view of one implementation of the present invention;
Figure 2 is an exploded view of the components of the implementation of figure 1;
Figure 3A to 3D are cross-sectional views of a device at various stages of operation;
Figure 4 is a detailed isometric view of the blood collection unit;
Figure 5 is a detailed cross sectional view illustrating the catch operation for the activation button;
Figure 6 is a view of the blood collection unit from the opposite end to figure 4;
Figure 7 is a detailed cross-sectional view illustrating the pivot of the blood collection unit;
Figure 8 is a view, partly in section, illustrating the operation of the interlock member before the lancet is actuated;
Figure 9 is a view similar to figure 8, showing the interlock after lancet actuation; and
Figures 10 and 11 illustrate a fluid unit, in a full and discharged condition.

### Detailed Description

The present invention will be described with reference to a particular implementation. It should be understood that the implementation discussed is purely illustrative, and is in no way limitative of the scope of the inventions disclosed. Various inventive features are disclosed, and it will be understood that this disclosure includes them in the combination as discussed, as well in in their individual integers and in subcombinations.

It should be appreciated that the present invention in one form extends to a complete test device, including a test material, such as a test strip. The test material may be in any form and may be intended to be read by the user either directly, for example via conventional line indications, or using an automated or partly automated reading device. The examples described relate primarily to a conventional lateral flow test strip, however, the invention may be employed with alternative test materials, for example other strip based tests, or electronic sensors. The present invention is not limited to any particular test, condition, test approach or test reading process. Aspects of the present invention are concerned, rather, with various usability, mechanical and fluid handling aspects, not the biochemistry or other aspects of the test material as such.

However, the present invention also extends to a device which does not include the test material, but is adapted to do so. Where the context admits, the description and claims should be read to include both a device with a test strip or other material in place, and a device in which the test strip or other material has not yet been inserted. Thus, the present invention extends to both devices with an integrated test material, and devices which have yet to have a test component inserted.

The present invention contemplates that a test fluid is dispensed onto the test material. This may be a suitable buffer, or any other fluid which it is desirable or necessary to dispense onto the test material, in order to facilitate the test. It will be appreciated that the nature and volume of test fluid will be dependent upon the test to be performed, and it is anticipated that the nature of the test fluid and volume will be selected to complement the intended test and test material. In some cases the test fluid may be an active component of the test, and not merely a buffer or carrier.

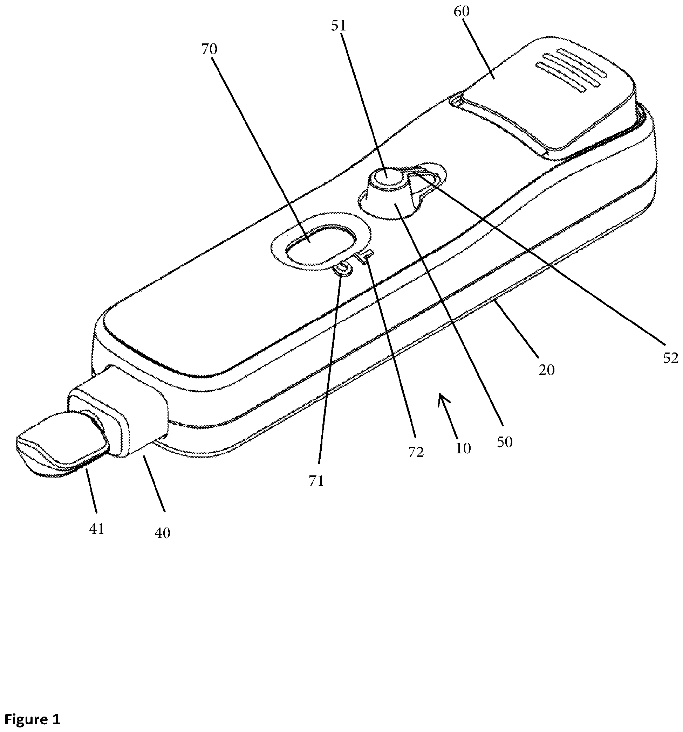

Figure 1 illustrates a device 10 according to one implementation of the present invention. It includes an upper shell 30, lower shell 20, blood collection unit 50 with blood collection recess 51 and channel 52. Device 10 further includes actuator 60, lancet mechanism 40 with cap 41, and result window 70.

The operation of the device, from perspective of the user, will now be described. This will be in the context of a self-test situation. However, it will be appreciated that many of the features and advantages of the illustrated device are also advantageous in point of care or other applications, and the scope of the invention includes self tests, professional use and point of care applications.

First, a user washes their hands, ideally with warm water to increase blood flow, and massages the selected finger for 10 seconds. The user then removes the lancet cap 41, by twisting and pulling. The user then presses hard on the exposed lancet mechanism 40. This releases a biased lancet which penetrates the user's finger, and then retracts within the housing of mechanism 40.

The user then milks the finger, so as to produce droplet of blood. Typically, 5 to 20 µl are required. The user places their finger against the blood collection recess 52, so that blood is collected. The blood flows into the recess 52. This provides a visual indication to the user that sufficient blood has been delivered to provide a valid test.

The next step is to depress actuator 60. As will be explained in more detail, until the lancet has been operated, an interlock prevents the actuator from being depressed. Activating the actuator causes the blood collection unit 50 to pivot slightly downward, so that the channel 52 contacts the test material 80 (not visible here) and the blood is delivered. At the same time, actuator 60 also depresses a reservoir of test fluid 100 (not visible here) and this is also delivered to the test material 80. A latch mechanism hold the actuator in the engaged position, so that the fluid is delivered.

A further feature of device 10 is the result window 70. Conventionally, a window is provided in a test device to show two indicator lines, for test outcome and a control line to indicate that the test is valid. These are conventionally open. According to the present implementation, a solid, partly transparent cover is provided over the test and control indicators. This prevents the user inadvertently putting the blood sample into the wrong opening. After the period appropriate for the test, the test and control lines are visible through the results window 70.

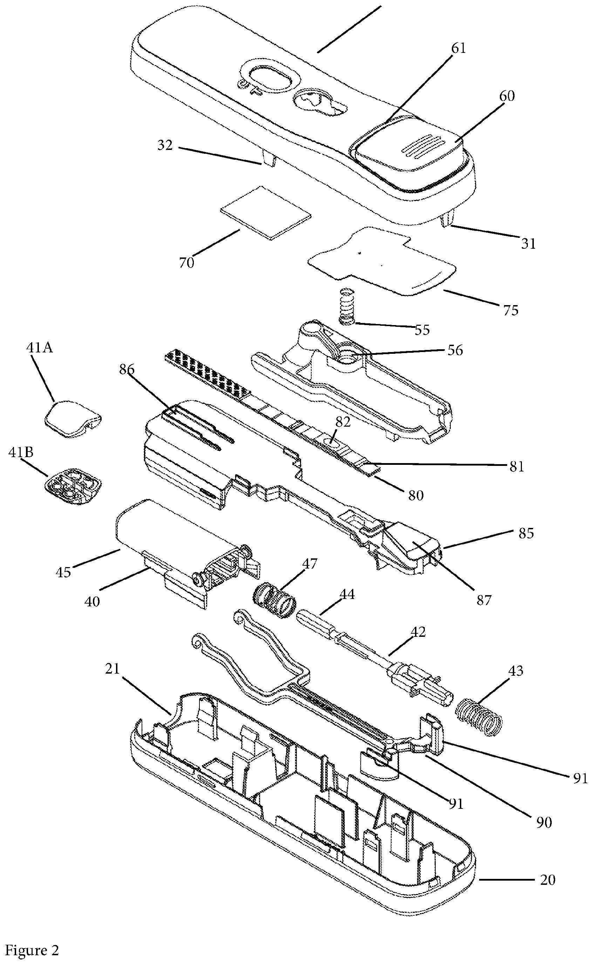

The structure and mechanism of device 10 will be further understood by reference to figure 2. Lower shell 20 includes various lugs and location features, including an opening 21 for the lancet mechanism 40. Next is the interlock 90, whose operation will be described below in more detail.

Lancet mechanism 40 is conventional in operation, and any suitable lancet mechanism could be substituted as will be apparent to those skilled in the art. Lancet 42 is biased by spring 43, so that when lancet housing 45 is sufficiently depressed relative to device 10, tabs break away and the lancet 42 projects and then return spring 47 retracts it for safety. Safety cap 44 is increased in usability by the addition of safety cover 41, formed as halves 41A, 41B.

Next in the assembly is strip carrier 85, on which strip 80 rests in use and is retained in the correct position for testing, using retainer 86.

Next is blood collection unit 50, including lug 56 and spring 55, which allow for the positive operation of the pivot function when the actuator is released, as will be described below.

Results window 70 as discussed above is transparent, so that the control and test lines can be seen. Preferably, the housing adjacent to the results window has markings of T and C adjacent to the potential results lines, indicating test and control.

Actuator 10 also compresses plate 75, which in turn compresses test fluid test fluid reservoir 100, not shown in this illustration, against surface 87. Upper shell 30 includes actuator 60, forms with a live hinge 61 to allow it to pivot downwards.

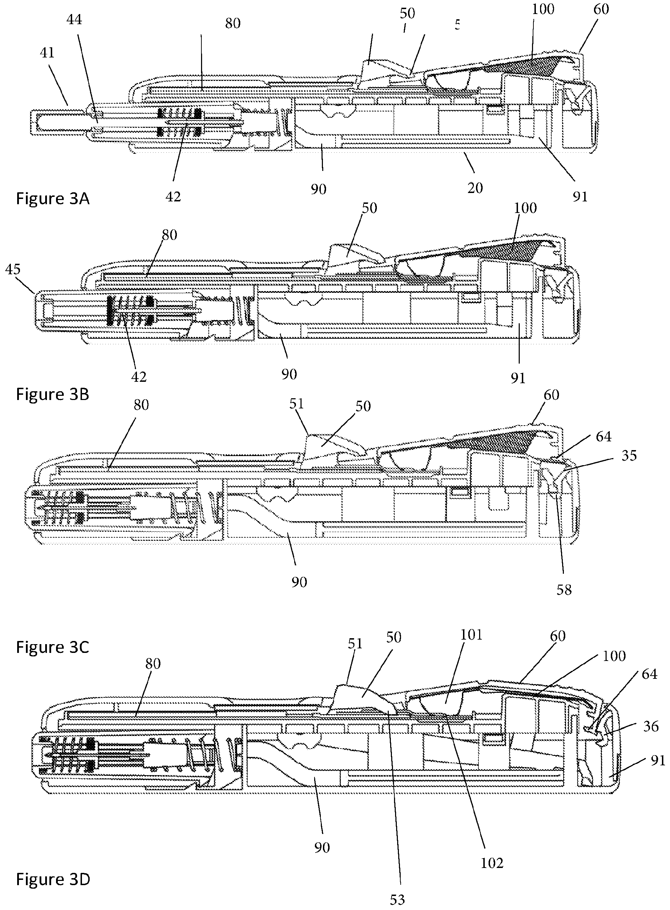

Figures 3A, 3B, 3C and 3D illustrate the operation of the various components of device 10, in cross section, at different points of operation of device 10. It is important to understand that the device described is a single use device, intended for a single test. Once the device has passed a stage in operation, for example the lancet has been fired, then it is not possible to return to an earlier stage and re-use the lancet.

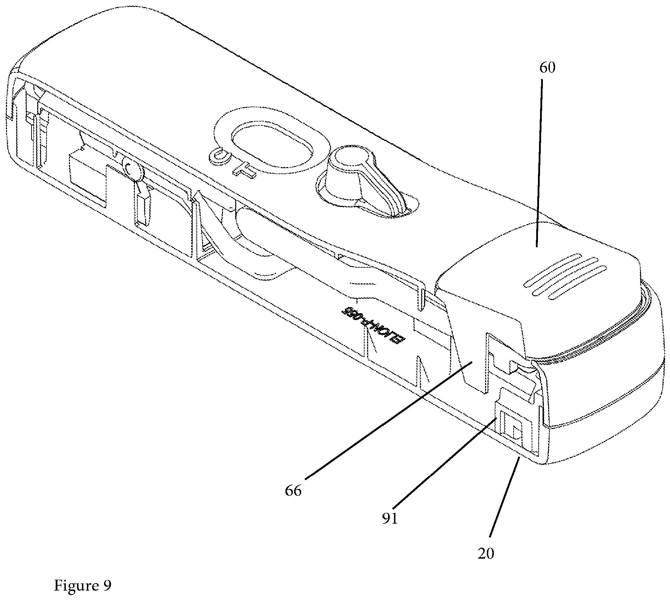

In Figure 3A, the device is as supplied, ready for use. The safety cap 44 is in position and lancet 42 has not been operated. Blood collection unit 50 is positioned so that its lower edge 53 is not in contact with the test strip 80. Reservoir 100 is filled with test fluid, for example buffer or any other fluid required to facilitate the test. Actuator 60 is not depressed. The interlock member 90 is located engaged with the lancet mechanism 40, and is slidable on the lower shell 20. At this stage, it is in the (in this view) left hand position, and the lock posts 91 are positioned to engage actuator posts 66 (not visible here, see figure 9). Thus, the user is prevented from depressing the actuator, and so neither blood nor test fluid can be delivered to test strip 80.

As a result, the user has limited opportunities to make erroneous use of the device. The accompanying instructions next tell the user to remove the safety cap 44.

Figure 3B illustrates device 10 with the safety cap 44 removed. The lancet is now ready for use. At this stage, nothing else in the device is altered. The user presses front surface 45 against the prepared finger, the lancet 42 fires, penetrates the finger, and retracts into the lancet mechanism 40. This is the situation shown in figure 3C.

The lancet mechanism is now in the retracted state, with front surface 45 moved back into lower shell 20, and the lancet 42 safely stored. Additionally, the retraction has pushed interlock member 90 to the right, and so moved lock posts 91 (only one is visible) to the right, so that they no longer prevent the movement of actuator 60.

The user at this stage should produce a droplet of blood, and place it into contact with blood collection recess 51. The user receives a visual indication that sufficient blood is provided when groove 52 is filled. Device 10 is now ready for the actuator to be depressed.

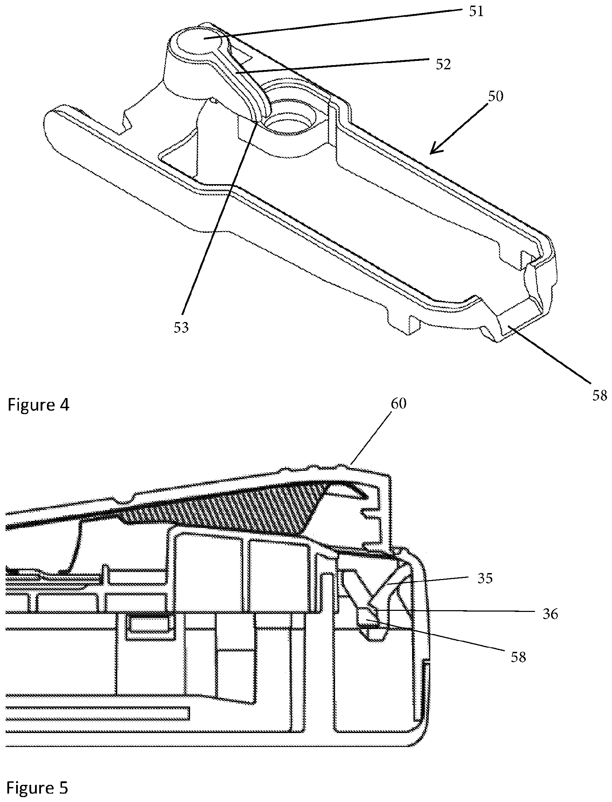

It is important to understand the mechanism of the blood collection unit. It is as this stage captured in a locked, upward position, biased by spring 55 on lug 56. It is also, via transverse section 58, captured in groove 36 of latch 35. Thus, at this stage the blood collection unit 50 is held away from contact with test strip 80. This can be seen in more detail in figure 5. A more detailed view of blood collection unit 50 can be seen in figure 4.

The user next depresses actuator 60. This has several consequences. The reservoir 100 is located directly under the actuator, so as to be depressed and the test fluid discharged into well 101 for discharge via opening 102 onto test strip 80. Thus, test fluid is discharged onto the test strip 80.

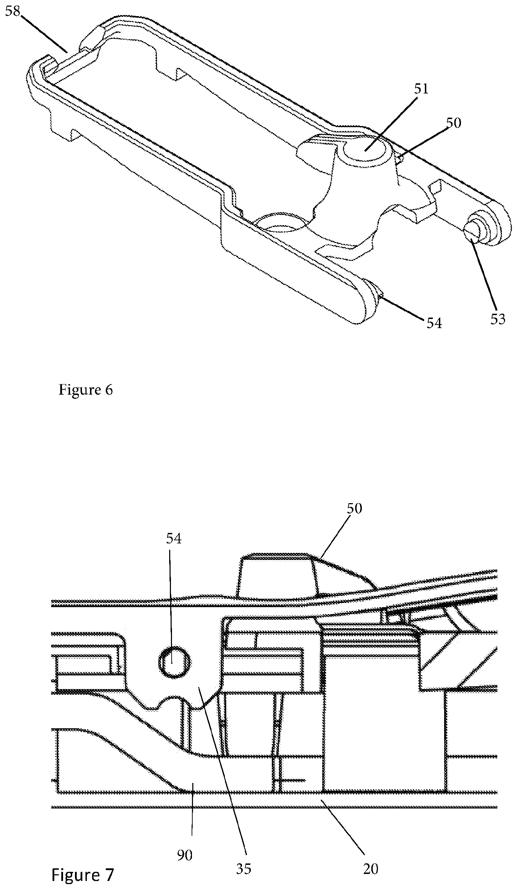

The actuator moving down also releases transverse section 58 from groove 36, and spring 55 then forces the blood collection unit 50 to pivot downwards, about pivot pins 53, 54. Figure 6 provides a more detailed view of the blood collection member 50 in which the pivot pins 53, 54 can be seen. These are located in holders 35 extending from top shell 30, one of which is visible in the detailed view of figure 7.

As a result of the pivoting movement, the lower edge 53 engages the test strip 80, and blood flows by capillary action from groove 52 into the bibulous material of the test strip 80.

The actuator 60 is further locked into the closed position. Latch 35 via recess 36 now engages the lower projection 64 of actuator 60, so that it is retained in the closed position. The retention assists in producing positive pressure within the reservoir 100 and well 101, so that the fluid is forced from reservoir 100 and into the test strip 80, so as to minimise surface losses of the test fluid and provide reliable delivery to the test strip 80.

The user then waits the appropriate time, and determines (in this case) if lines appear in the test and control windows. The test result can thereby be determined, together with verification that a valid test has been conducted.

It is generally preferable that the blood is always delivered at the same time, or earlier, than the test fluid, particularly if the fluid is a buffer. The purpose of the buffer is to provide enough fluid to sufficiently drive the movement of the blood along the strip. If the buffer is added before the blood, or runs ahead of the blood, the test may not function correctly.

In one respect, the test fluid is added at about area 81 on the test strip, and blood is delivered from blood collection unit 50, because the action of spring 55, at about area 82. Hence, even if the blood and buffer are added at the same time, the buffer is behind the blood.

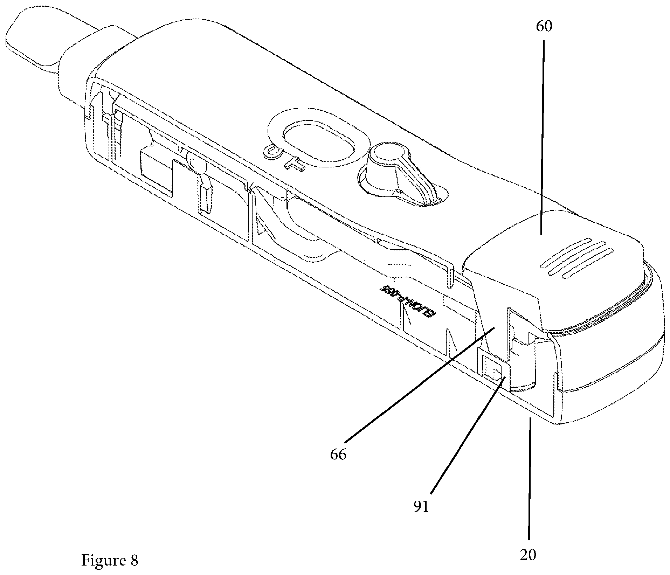

Figures 8 and 9 illustrate the operation of the interlock member 90 in more detail. Figure 8 corresponds to the situation in figure 3A, in which device 10 is ready for use, but the lancet 42 has not been prepared or fired. Lock post 91 is at it's original, left-most position, that is, closest to the lancet end. It will be understood that another lock post is located on the other side of the lower shell, as can be seen in figure 2. In this position, lock post 91 engages actuator post 66, so that actuator 60 cannot be depressed.

Figure 9 llustrate the situation in figure 3C, after the lancet has been fired. Lock post 91 has now moved to the right, that is, to its position at the end closest to the actuator 60. The lock posts 91 no longer engage the actuator posts 66 and actuator 60 can be depressed.

The arrangement illustrated has the actuator at the opposite end to the lancet. When the device is used for self test, this is convenient for the user, as they lance their finger at one end, place the blood onto the blood recess 51, and operate the actuator from the other end. The interior structure is better organised that in arrangements in which the actuator, reservoir and lancet were all at the same end.

A further advantageous feature is that the actuator brings the blood into contact with the test strip in a controlled way, in which the movement is controlled by the device and is not dependant upon the force used by the user. The actuator simply releases the blood collection unit, no understanding or other control is required by the user.

Similarly, the more or less simultaneous release of test fluid and blood, preferably each onto their respective contact areas on the strip, means that the user need not worry about sequencing or volume control, or even timing - the device performs these tasks from a single actuator operation.

The reservoir unit is preferable formed and constructed as disclosed in more detail in PCT/AU2016/051134. While this is a suitable implementation, the present invention is not limited to a fluid delivery mechanism constructed in this manner.

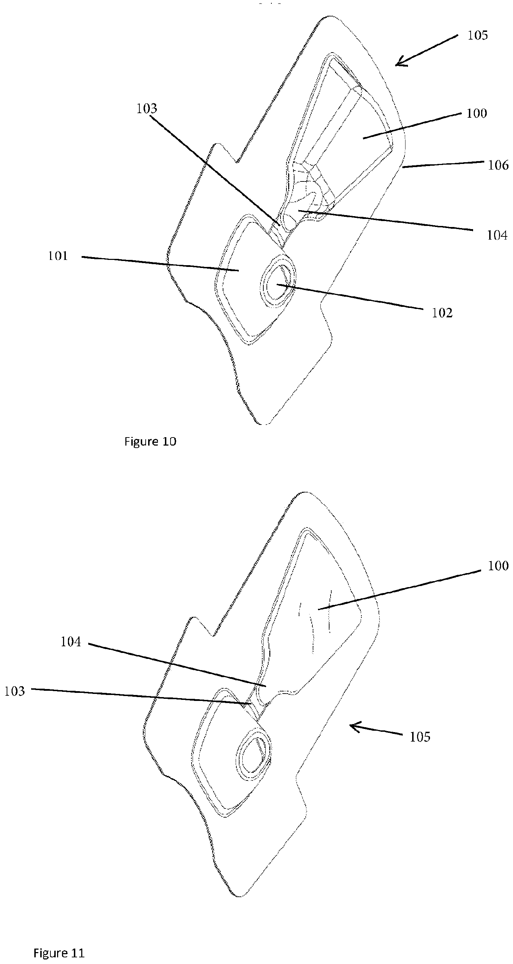

Figures 10 and 11 illustrate respectively a full and a discharged fluid unit 105. The unit includes a reservoir 100, a well 101 with a discharge opening 102, and a conduit 104 connecting reservoir 100 to well 101. Conduit 104 is sealed by a frangible seal, which ruptures operatively under pressure from the actuator 60. The reservoir is forced to close and forces fluid into well 101 and then through opening 102, which is in contact with the test strip 80.

The fluid unit 105 may be conveniently formed from laminated heat sealable material, with a relatively stronger seal around the periphery and overlapping edges 106, with the weaker, frangible seal 103, preferably formed as per the above referenced application.

The size of the fluid unit, as well as the capacity of the blood collecting unit, may be varied by appropriate dimensioning to suit the requirements of the test. It will be apparent that within the size constraints of the device, a variety of size options can be accommodated, so that the same basic device may be varied in those component only to provide volumes suitable for different tests.

The present invention may be implemented including electronic components, for example an automated reader for a lateral flow test, or an electronic test material. In such an implementation, the actuator may additionally serve to activate the electronics, for example using a projection to operate a switch to close a circuit.

## Claims

1. An integrated blood test unit (10) including a blood collection unit (50), a test component (80), a reservoir (100) containing test fluid, and an actuator (60), wherein operatively activation of the actuator (60) causes movement of the blood collection unit (50) to deliver blood to the test component (80), and activation of the actuator (60) further causes the test fluid to be released from the reservoir (100) in order to allow the test fluid to contact the test component.

2. An integrated blood test unit (10) according to claim 1, wherein in a sample collection position the blood collection unit (50) is biased towards the test component (80) but prevented from delivering the blood to the test component (80) by a latch (35), and wherein operatively, activation of the actuator (60) releases the latch (35) and allows the blood collection unit (50) to move to a sample delivery position, so that blood is delivered to the test component (80).

3. An integrated blood test unit (10) according to claim 1 or claim 2, wherein in response to activation of the actuator (60), the blood collection unit (50) tilts into the sample delivery position so as to deliver the blood to the test component (80).

4. An integrated blood test unit (10) according to any one of the preceding claims, wherein the test unit (10) further includes an electronic component, and the electronic component is made operative by activation of the actuator (60).

5. An integrated blood test unit according to any one of the preceding claims, wherein a retainer (35) is provided, so that operatively, after the actuator (60) is activated, the actuator (60) is locked into an actuated position.

6. An integrated blood test unit (10) according to claim 5, wherein the locked actuator (60) exerts a continuing force so as to compress the reservoir (100) in order to dispense the test fluid onto the test component (80).

7. An integrated test unit (10) according to any one of the preceding claims, wherein the test unit (10) further provides an integrated lancet (42), and an interlock is provided so that in operation, the actuator (60) is inoperative, and so the blood collection unit (50) cannot move to a delivery position, and the test fluid cannot be released, until after the lancet (42) has been discharged.

8. An integrated blood test unit (10) according to any one of the preceding claims, wherein the test unit (10) further includes a transparent window (70) located so that the or each visual indication by the test component (80) is visible.

9. An integrated blood test unit (10) according to any one of the preceding claims, further including an integrated lancet (42), wherein the actuator (60) and the integrated lancet (42) are located at opposite ends on the integrated test unit, so that the operation of the lancet (42) is convenient for a self test relative to the actuator(60).

10. An integrated blood test unit (10) according to any one of the preceding claims, wherein the blood collection unit (50) includes a blood collection recess (51), the recess (51) being adapted to directly receive blood from a user, the recess (51) including a channel (52) which operatively is adapted to progressively fills with blood, such that when the channel (52) is full it provides a visual indication to the user that sufficient blood has been received to conduct a test

11. An integrated blood test unit (10) according to claim 10, wherein in operation the blood collection unit (50) is adapted to tilt downward to place an end of the channel (52) adjacent to the test component (80), so that blood flows from the channel (52) to the test component (80) .

12. An integrated blood test unit (10) according to claim 11, wherein the unit is arranged so that in operation the blood is delivered to the test component (80) at an area (82), and the test fluid is delivered to the test component (80) at a second area (81), so that the test fluid is delivered behind the blood to test component (80) so that the test fluid can drive the movement of blood for use in the test component (80).

## Patentansprüche

1. Integrierte Bluttesteinheit (10), beinhaltend eine Blutsammeleinheit (50), eine Testkomponente (80), ein Reservoir (100), das Testfluid enthält, und einen Aktor (60), wobei operativ eine Aktivierung des Aktors (60) eine Bewegung der Blutsammeleinheit (50) bewirkt, um Blut an die Testkomponente (80) abzugeben, und eine Aktivierung des Aktors (60) ferner bewirkt, dass das Testfluid aus dem Reservoir (100) freigegeben wird, um zu ermöglichen, dass das Testfluid mit der Testkomponente in Kontakt kommt.

2. Integrierte Bluttesteinheit (10) nach Anspruch 1, wobei in einer Probenentnahmeposition die Blutentnahmeeinheit (50) in Richtung der Testkomponente (80) vorgespannt ist, aber durch eine Verriegelung (35) daran gehindert wird, das Blut an die Testkomponente (80) zu liefern, und wobei operativ eine Aktivierung des Aktors (60) die Verriegelung (35) freigibt und es der Blutsammeleinheit (50) ermöglicht, sich in eine Probenabgabeposition zu bewegen, sodass Blut an die Testkomponente (80) abgegeben wird.

3. Integrierte Bluttesteinheit (10) nach Anspruch 1 oder Anspruch 2, wobei sich die Blutsammeleinheit (50) als Reaktion auf eine Aktivierung des Aktors (60) in die Probenabgabeposition neigt, um das Blut an die Testkomponente (80) abzugeben.

4. Integrierte Bluttesteinheit (10) nach einem der vorhergehenden Ansprüche, wobei die Testeinheit (10) ferner eine elektronische Komponente beinhaltet und die elektronische Komponente durch Aktivierung des Aktors (60) in Betrieb gesetzt wird.

5. Integrierte Bluttesteinheit nach einem der vorhergehenden Ansprüche, wobei ein Rückhalteelement (35) bereitgestellt ist, sodass, nachdem der Aktor (60) aktiviert ist, der Aktor (60) operativ in einer betätigten Position verriegelt wird.

6. Integrierte Bluttesteinheit (10) nach Anspruch 5, wobei der verriegelte Aktor (60) eine fortgesetzte Kraft ausübt, damit das Reservoir (100) komprimiert wird, um das Testfluid auf die Testkomponente (80) auszugeben.

7. Integrierte Testeinheit (10) nach einem der vorhergehenden Ansprüche, wobei die Testeinheit (10) ferner eine integrierte Lanzette (42) bereitstellt, und eine Sperre bereitgestellt ist, sodass im Betrieb der Aktor (60) außer Betrieb gesetzt ist, sodass sich die Blutsammeleinheit (50) nicht in eine Abgabeposition bewegen kann, und das Testfluid nicht freigegeben werden kann, bevor die Lanzette (42) ausgestoßen wurde.

8. Integrierte Bluttesteinheit (10) nach einem der vorhergehenden Ansprüche, wobei die Testeinheit (10) ferner ein transparentes Fenster (70) beinhaltet, das so positioniert ist, dass die oder jede visuelle Angabe durch die Testkomponente (80) sichtbar ist.

9. Integrierte Bluttesteinheit (10) nach einem der vorhergehenden Ansprüche, ferner beinhaltend eine integrierte Lanzette (42), wobei der Aktor (60) und die integrierte Lanzette (42) an gegenüberliegenden Enden an der integrierten Testeinheit positioniert sind, sodass der Betrieb der Lanzette (42) für einen Selbsttest relativ zu dem Aktor (60) geeignet ist.

10. Integrierte Bluttesteinheit (10) nach einem der vorhergehenden Ansprüche, beinhaltend eine Blutsammeleinheit (50), die eine Blutsammelvertiefung (51) beinhaltet, wobei die Vertiefung (51) dazu angepasst ist, Blut von einem Benutzer direkt aufzunehmen, wobei die Vertiefung (51) einen Kanal (52) beinhaltet, der operativ dazu angepasst ist, sich fortschreitend mit Blut zu füllen, sodass, wenn der Kanal (52) voll ist, er eine visuelle Angabe für den Benutzer bereitstellt, dass ausreichend Blut aufgenommen wurde, um einen Test durchzuführen.

11. Integrierte Bluttesteinheit (10) nach Anspruch 10, wobei im Betrieb die Blutsammeleinheit (50) dazu angepasst ist, nach unten zu kippen, um ein Ende des Kanals (52) benachbart der Testkomponente (80) zu platzieren, sodass Blut von dem Kanal (52) zu der Testkomponente (80) fließt.

12. Integrierte Bluttesteinheit (10) nach Anspruch 11, wobei die Einheit so angeordnet ist, dass im Betrieb das Blut an einem Bereich (82) an die Testkomponente (80) abgegeben wird und das Testfluid an einem zweiten Bereich (81) an die Testkomponente (80) abgegeben wird, sodass das Testfluid hinter dem Blut an die Testkomponente (80) abgegeben wird, sodass das Testfluid die Bewegung von Blut zur Verwendung in der Testkomponente (80) antreiben kann.

## Revendications

1. Unité de test sanguin intégrée (10) comportant une unité de collecte de sang (50), un composant de test (80), un réservoir (100) contenant un fluide de test, et un actionneur (60), dans laquelle, de manière fonctionnelle, l'activation de l'actionneur (60) provoque le déplacement de l'unité de collecte de sang (50) pour acheminer du sang vers le composant de test (80), et l'activation de l'actionneur (60) provoque en outre la libération du fluide de test du réservoir (100) afin de permettre au fluide de test d'entrer en contact avec le composant de test.

2. Unité de test sanguin intégrée (10) selon la revendication 1, dans laquelle, dans une position de collecte d'échantillon, l'unité de collecte de sang (50) est sollicitée vers le composant de test (80) mais empêchée d'acheminer le sang au composant de test (80) par un verrou (35), et dans laquelle, de manière fonctionnelle, l'activation de l'actionneur (60) libère le verrou (35) et permet à l'unité de collecte de sang (50) de se déplacer vers une position d'acheminement d'échantillon, de sorte que le sang est acheminé au composant de test (80).

3. Unité de test sanguin intégrée (10) selon la revendication 1 ou la revendication 2, dans laquelle, en réponse à l'activation de l'actionneur (60), l'unité de collecte de sang (50) bascule dans la position d'acheminement d'échantillon de manière à acheminer le sang vers le composant de test (80).

4. Unité de test sanguin intégrée (10) selon l'une quelconque des revendications précédentes, dans laquelle l'unité de test (10) comporte en outre un composant électronique, et le composant électronique est rendu fonctionnel par l'activation de l'actionneur (60).

5. Unité de test sanguin intégrée selon l'une quelconque des revendications précédentes, dans laquelle un dispositif de retenue (35) est prévu, de sorte que, de manière fonctionnelle, après l'activation de l'actionneur (60), l'actionneur (60) est verrouillé dans une position actionnée.

6. Unité de test sanguin intégrée (10) selon la revendication 5, dans laquelle l'actionneur verrouillé (60) exerce une force continue de manière à comprimer le réservoir (100) pour acheminer le fluide de test sur le composant de test (80) .

7. Unité de test sanguin intégrée (10) selon l'une quelconque des revendications précédentes, dans laquelle l'unité de test (10) fournit en outre une lancette intégrée (42), et un verrouillage est prévu de sorte que, lors du fonctionnement, l'actionneur (60) est inopérant, et de sorte que l'unité de collecte de sang (50) ne peut pas se déplacer vers une position d'acheminement, et le fluide de test ne peut pas être libéré, jusqu'à ce que la lancette (42) ait été déchargée.

8. Unité de test sanguin intégrée (10) selon l'une quelconque des revendications précédentes, dans laquelle l'unité de test (10) comporte en outre une fenêtre transparente (70) située de manière à ce que la ou chaque indication visuelle par le composant de test (80) soit visible.

9. Unité de test sanguin intégrée (10) selon l'une quelconque des revendications précédentes, comportant en outre une lancette intégrée (42), dans laquelle l'actionneur (60) et la lancette intégrée (42) sont situés à des extrémités opposées sur l'unité de test intégrée, de sorte que le fonctionnement de la lancette (42) convient pour un auto-test par rapport à l'actionneur (60).

10. Unité de test sanguin intégrée (10) selon l'une quelconque des revendications précédentes, comportant une unité de collecte de sang (50) comportant un évidement de collecte de sang (51), l'évidement (51) étant conçu pour recevoir directement du sang d'un utilisateur, l'évidement (51) comportant un canal (52) qui est agencé de manière fonctionnelle pour se remplir progressivement de sang, de telle sorte que lorsque le canal (52) est plein, il fournit une indication visuelle à l'utilisateur qu'une quantité suffisante de sang a été reçue pour réaliser un test.

11. Unité de test sanguin intégrée (10) selon la revendication 10, dans laquelle, lors du fonctionnement, l'unité de collecte de sang (50) est conçue pour basculer vers le bas pour placer une extrémité du canal (52) adjacente au composant de test (80), de sorte que le sang s'écoule du canal (52) vers le composant de test (80).

12. Unité de test sanguin intégrée (10) selon la revendication 11, dans laquelle l'unité est agencée de sorte que, lors du fonctionnement, le sang peut être acheminé au composant de test (80) au niveau d'une zone (82), et le fluide de test est acheminé au composant de test (80) au niveau d'une seconde zone (81), de sorte que le fluide de test est acheminé derrière le sang vers le composant de test (80), de sorte que le fluide de test peut entraîner le mouvement du sang destiné à être utilisé dans le composant de test (80).
